# EUROPÄISCHE PATENTANMELDUNG

(11) **EP 2 821 705 A1**
(43) Veröffentlichungstag der Anmeldung: **07.01.2015**
(21) Anmeldenummer: 14174970.5
(22) Anmeldetag: 30.06.2014
(51) Int. Cl.: F23N 1/02, F23N 5/00, F23N 5/12, G01N 25/20, G01N 25/24, G01N 33/22

(54) **Verfahren und Vorrichtung zur Bestimmung des Brennwerts einer Brenngasmischung mittels eines Ionisationssensors**

(30) Priorität: 03.07.2013 DE 102013106987
(71) Anmelder: Karl Dungs GmbH & Co.KG, 73660 Urbach (DE)
(72) Erfinder: Petermann, Harald, 73669 Lichtenwald (DE); Berger, Siegfried, 73278 Schlierbach (DE); Schmidt, Oliver, 74196 Neuenstadt (DE); Haug, Rudolf, 26689 Apen (DE)
(74) Vertreter: Rüger, Barthelt & Abel

(57) **Zusammenfassung**

Die Erfindung betrifft ein Verfahren und eine Vorrichtung zur Bestimmung einer den Brennwert eines gasförmigen Brennstoffes (G) beschreibenden Brennwertgröße (H). Die Erfindung betrifft auch eine gasbetriebene Einrichtung (40), die ein erfindungsgemäßes Verfahren bzw. eine erfindungsgemäße Vorrichtung (10) verwendet. Die Vorrichtung (10) verfügt über einen Testbrenner (11) mit einer Testbrennkammer (12). Ein Luftzahlsensor (24) ist in einem Abgaskanal (22) des Testbrenners (11) angeordnet und misst ein Luftzahlsignal (SL), das der Luftzahl (λ) des Abgases entspricht. Über eine Teststeuereinheit (25) wird abhängig vom empfangenen Luftzahlsignal SL wenigstens ein Stellsignal (S1, S2) für eine Testzuführeinheit (15) erzeugt. Das wenigstens eine Stellsignal (S1, S2) steuert die Menge und/oder den Anteil eines gasförmigen Brennstoffs (G) bzw. eines sauerstoffhaltigen Gases (L), das der Testbrennkammer (12) zugeführt wird. In der Testbrennkammer (12) verbrennt bzw. oxidiert das Gemisch aus Brennstoff (G) und dem sauerstoffhaltigen Gas (L). In der Brennkammer (12) ist eine Brennwertsensoranordnung (30) vorhanden. Zu dieser gehört zumindest ein Ionisationssensor (31) und vorzugsweise auch ein Temperatursensor (33). Das wenigstens eine Sensorsignal (I bzw. ST) der Brennwertsensoranordnung wird einer Ermittlungseinheit 32 übermittelt. Dort wird vorzugsweise anhand eines Kennfeldes aus diesen Größen eine Brennwertgröße (H) ermittelt.

## Beschreibung

Die Erfindung betrifft ein Verfahren und eine Vorrichtung zur Bestimmung einer Brennwertgröße für den Brennwert eines gasförmigen Brennstoffs. Die Erfindung betrifft auch eine gasbetriebene Einrichtung mit einer Vorrichtung zur Bestimmung der Brennwertgröße.

Brenner von gasbetriebenen Einrichtungen werden in der Regel so eingestellt, dass eine vollständige Verbrennung eines zugeführten gasförmigen Brennstoffes erfolgt. Hierfür wird die Menge an zugeführtem Brennstoff sowie die Menge an einem zugeführten sauerstoffhaltigen Gas, insbesondere Luft, entsprechend eingestellt. In der Regel ist im Abgaskanal eines solchen Brenners ein Sensor zur Bestimmung der Luftzahl vorhanden, so dass eine stöchiometrische oder überstöchimetrische Verbrennung erfolgt.

Ein gasförmiger Brennstoff, beispielsweise Biogas, Erdgas, Wasserstoff oder ein Gasgemisch aus mehreren dieser Gase weist unterschiedliche Brennwerte auf. Gasbetriebene Einrichtungen müssen häufig mit unterschiedlichen Gasen bzw. Gasgemischen betrieben werden können, die während des Betriebs auch variieren können. So schwankt der Anteil von Methan im Erdgas in der Regel zwischen 75% und etwa 95% bis 98%. Außerdem können im Erdgas unterschiedliche Anteile von Ethan, Propan, Butan, Stickstoff oder Kohlendioxid vorhanden sein. Wie bereits erwähnt, können auch Gasgemische als gasförmiger Brennstoff verwendet werden, so dass zusätzlich Biogas oder Biogasanteile sowie Wasserstoff oder Wasserstoffanteile vorhanden sein können. Der Brennwert variiert dabei entsprechend.

Zum Betreiben einer gasbetriebenen Einrichtung kann es daher vorteilhaft sein, wenn die Einstellung abhängig vom verwendeten Gas bzw. Gasgemisch vorgenommen werden kann.

Zu diesem Zweck wurden verschiedenen Vorrichtungen und Systeme vorgeschlagen. Beispielsweise beschreibt EP 199 55 76 A1 eine Einrichtung mit einem Fabry-Perot-Interferometer zur Bestimmung von Stoffen bzw. Stoffkonzentrationen im gasförmigen Brennstoff. Die Einrichtung nach DE 103 02 487 A1 verwendet ein Infrarot-Absorptions-Gasmesssystem mit zumindest zwei Messkanälen für Kohlendioxid und Alkale, um eine Brenngaszusammensetzung zu bestimmen.

In US 411 81 72 A ist vorgeschlagen, einen Testbrenner mit einem Luftzahlsensor zur Einstellung einer stöchiometrischen Verbrennung zu verwenden und diese Einstellung dann auf einen Hauptbrenner zu übertragen. Eine Brennwertgröße wird hier nicht ermittelt.

Eine Brennwertbestimmung in Abhängigkeit von der Messung der Schallgeschwindigkeit bei verschiedenen Temperaturen ist in DE 699 24 828 T2 beschrieben.

Eine Brennwertermittlung erfolgt gemäß DE 101 298 08 dadurch, dass ein Brennstoff-Luft-Gemisch verbrannt und anschließend gekühlt wird. Dabei wird die Temperatur an verschiedenen Stellen gemessen und ein Wärmestrom berechnet, anhand dem der Heizwert ermittelt werden kann.

Einen ähnlichen Ansatz verfolgt das in DE 100 10 291 A1 vorgeschlagene Verfahren. Dort wird ein Wärmeübertragungselement in die Brennkammer eingesetzt und soviel Wärme vom Wärmeübertragungselement abgezogen, dass eine konstante Temperatur des Wärmeübertragungselementes erreicht ist. Daraus wird dann geschlossen, dass die vom Brenner erzeugte Wärmeleistung der Wärmeleistung entspricht, die vom Wärmeübertragungselement abgezogen wurde. Aus dieser Wärmeleistung wird dann der Brennwert ermittelt.

Ein weiteres Verfahren und eine Vorrichtung zur Bestimmung des Wobbe-Index bzw. des Brennwertes ist in EP 1 770 390 A1 beschrieben. Dort sind zwei Heizplatten in einer Kammer angeordnet. Die zweite Heizplatte dient dabei als katalytische Heizplatte. Über eine Kontrolleinrichtung wird die Temperatur zumindest einer der beiden Heizplatten konstant gehalten. Strömt das Gas-Luft-Gemisch an der ersten Heizplatte vorbei, kann die Leistung bestimmt werden, die notwendig ist, um diese Heizplatte auf einer konstanten Temperatur zu halten. Im Bereich der anderen Heizplatte kann das Gas-Luft-Gemisch verbrannt werden. Es wird ebenfalls erfasst, welche Leistung notwendig ist, um auch die zweite Heizplatte auf einer konstanten Temperatur zu halten. Aus diesen Leistungswerten zur Temperaturregelung kann dann ein Brennwert ermittelt werden.

Ausgehend hiervon kann es als Aufgabe der vorliegenden Erfindung angesehen werden, eine Vorrichtung und ein Verfahren zu schaffen, wobei die Vorrichtung mit einfachen Mitteln auskommt und eine schnelle und sichere Brennwertermittlung ermöglicht.

Erfindungsgemäß wird hierfür ein Testbrenner mit einer Testbrennkammer verwendet. Aus der Testbrennkammer führt ein Abgaskanal heraus, in dem ein Luftzahlsensor angeordnet ist. Der Luftzahlsensor liefert ein Luftzahlsignal, das die Luftzahl im Abgas der Testbrennkammer angibt. Das Luftzahlsignal wird einer Teststeuereinheit übermittelt. Die Teststeuereinheit erzeugt abhängig vom Luftzahlsignal wenigstens ein Stellsignal. Eine Testzuführeinheit dient dazu, den gasförmigen Brennstoff gemeinsam mit einem sauerstoffhaltigen Gas, beispielsweise Luft, der Testbrennkammer zuzuführen. Die Testzuführeinheit ist steuerbar und kann dadurch den Anteil und/oder die Menge des in die Testbrennkammer zugeführten gasförmigen Brennstoffs und/oder des in die Testbrennkammer eingeleiteten sauerstoffhaltigen Gases einstellen. Dabei wird der Volumenstrom oder der Massenstrom des sauerstoffhaltigen Gases und/oder des gasförmigen Brennstoffes durch die Testzuführeinheit abhängig von dem wenigstens einen Stellsignal eingestellt. Dadurch kann eine Verbrennung bzw. eine Oxidation des Gemischs aus dem Brennstoff und dem sauerstoffhaltigen Gas so eingestellt werden, dass die Luftzahl einem vorgegebenen Luftzahlsollwert entspricht.

Im Bereich der Flamme bzw. im Bereich der Oxidation des brennstoffhaltigen Gases in der Testbrennkammer ist eine Brennwertsensoranordnung vorhanden. Die Brennwertsensoranordnung weist wenigstens einen Ionisationssensor auf, der ein Ionisationssignal und insbesondere einen Ionisationsstrom erzeugt. Zu der Brennwertsensoranordnung können weitere Sensoren gehören, die beispielsweise wenigstens ein Temperatursensor und/oder wenigstens ein optischer Sensor.

Die Vorrichtung weist außerdem eine Ermittlungseinheit auf. Dieser Ermittlungseinheit wird das wenigstens eine Sensorsignal der Brennwertsensoranordnung und mithin das Ionisationssignal übermittelt. Abhängig vom Ionisationssignal wird eine Brennwertgröße ermittelt, die den Brennwert des gasförmigen Brennstoffes angibt. Das Ionisationssignal bzw. der Ionisationsstrom wird hierfür insbesondere bei wenigstens einem oder bei mehreren vorgegebenen Luftzahlwerten ermittelt. Die Ionisation in der Testbrennkammer bei einer vorgegebenen Luftzahl ist charakteristisch für den Heizwert. Daraus kann in der Ermittlungseinheit die Brennwertgröße abgeleitet werden.

Um die Genauigkeit der Ermittlung der Brennwertgröße zu verbessern können bei der Ermittlung der Brennwertgröße weitere Parameter bzw. Signale berücksichtigt werden, insbesondere eine oder mehrere der folgenden Größen:
- wenigstens eine die Brennerleitung charakterisierende Größe, beispielsweise das wenigstens eine Stellsignal zur Einstellung der Testzuführeinrichtung;
- ein Temperaturwert innerhalb des Testbrennkammer; hierfür kann die Brennwertsensoranordnung einen Temperatursensor aufweisen;
- eventuell ein Signal eines optischen Sensors der Brennwertsensoranordnung, mittels dem ein Spektrum oder ein Teil eines Spektrums des von der Flamme des Testbrenners abgestrahlten Lichts bestimmt wird.

Bevorzugt wird die Brennwertgröße in der Ermittlungseinheit aus dem Temperatursignal eines Temperatursensors, aus dem Ionisationssignal sowie dem wenigstens einen Stellsignal der Teststeuereinheit ermittelt. Über dieses Wertetripel kann eine ausreichend exakte Bestimmung der Brennwertgröße erfolgen, wobei die Vorrichtung mit sehr einfachen Mitteln auskommt. Weitere Sensortypen zur Spektralanalyse, zur optischen Analyse oder zur Messung der Schallgeschwindigkeit sind nicht notwendig. Da die Leistung des Testbrenners die Ionisation beeinflusst, kann insbesondere sehr einfach das ohnehin vorhandene wenigstens eine Stellsignal zur Bewertung der Brennerleistung verwendet werden. Denn daraus lässt sich die Menge an Brennstoff und/oder Luft, die in die Testbrennkammer eingeleitet wird, bestimmen. Auch andere die Brennerleistung charakterisierende Größen könnten alternativ ermittelt werden.

Die Ermittlungseinheit kann bei einer Ausführungsform einen Speicher aufweisen, in dem eine Ermittlungsvorschrift zur Bestimmung der Brennwertgröße abgespeichert ist. Beispielsweise kann als Ermittlungsvorschrift eine Tabelle, eine Funktion, ein Kennfeld oder ähnliches dienen. Um die notwendige Rechenleistung zu minimieren, ist vorzugsweise ein Kennfeld im Speicher hinterlegt, aus dem abhängig von den berücksichtigten Eingangssignalen bzw. Parametern die Brennwertgröße einfach und schnell während des Betriebs des Testbrenners ermittelt werden kann.

In der Testbrennkammer kann über eine Zündeinrichtung, beispielsweise eine Zündelektrode, eine Testflamme erzeugt werden. Alternativ kann auch eine Oxidation ohne Flamme erzeugt werden, beispielsweise mit Hilfe eines Katalysators. Vorzugsweise ist die Brennwertsensoranordnung im Bereich der Testflamme angeordnet.

Bei einem Ausführungsbeispiel kann die Brennwertsensoranordnung auch mehrere Ionisationssensoren aufweisen, die an verschiedenen Stellen innerhalb der Testbrennkammer angeordnet sind. Die Ionisation und mithin das Ionisationssignal hängen von der Position des Ionisationssensors im Bereich der Testflamme ab. Durch das Anordnen mehrerer Io-nisationssensoren an unterschiedlichen Stellen kann eine weitere Genauigkeitserhöhung bei der Ermittlung der Brennwertgröße erreicht werden.

Die Vorrichtung zur Bestimmung der Brennwertgröße kann Bestandteil einer gasbetriebenen Einrichtung sein. Ein Teil des für die gasbetriebene Einrichtung verwendeten gasförmigen Brennstoffes wird dem Testbrenner zugeführt und dessen Brennwert wie oben erläutert bestimmt.

Die gasbetriebene Einrichtung kann eine Hauptbrennkammer, eine Hauptzuführeinrichtung und eine Hauptsteuereinheit aufweisen. Die Hauptzuführeinrichtung dient dazu, die Menge des gasförmigen Brennstoffes und/oder die Menge des sauerstoffhaltigen Gases, die in den Hauptbrennkammer eingeleitet wird, einzustellen. Über die Hauptsteuereinheit wird wenigstens ein Hauptstellsignal zur Ansteuerung der Hauptzuführeinrichtung erzeugt. Dieses Hauptstellsignal wird in der Hauptsteuereinheit abhängig von der in der Vorrichtung zur Ermittlung der Brennwertgröße ermittelten Brennwertgröße erzeugt. Somit kann der Hauptbrenner der gasbetriebenen Einrichtung abhängig von der Brennwertgröße gesteuert und eingestellt werden.

Insbesondere wird der Betrieb des Hauptbrenners erst dann gestartet, bzw. ermöglicht, wenn vorher über die Vorrichtung eine Brennwertgröße für den aktuell verwendeten gasförmigen Brennstoff ermittelt wurde. Erst nachdem die Hauptsteuereinheit einen aktuellen Wert für die Brennwertgrößer erhält, steuert sie abhängig davon die Hauptzuführeinrichtung an und nimmt den Hauptbrenner in Betrieb. Dadurch können ungünstige Betriebsbedingungen beim Starten des Hauptbrenners vermieden werden. Abhängig von den Bestandteilen des gasförmigen Brennstoffes kann es beim Starten des Brenners bei zu fetten Gemischen auch zu explosionsartigen Zündungen und gefährlichen Betriebszuständen kommen. Dies wird erfindungsgemäße dadurch vermieden, dass über die Vorrichtung mit dem Testbrenner eine Brennwertgröße für den zugeführten gasförmigen Brennstoff vorliegt.

Vorzugsweise wird die Hauptzuführeinheit zusätzlich abhängig von einem Bediensignal eingestellt. Das Bediensignal kann manuell vom Bediener oder automatisch von einem Regler oder einer sonstigen Einrichtung vorgegeben werden. Über das Bediensignal kann die Leistung des Hauptbrenners eingestellt werden.

Es ist außerdem vorteilhaft, wenn der aktuell ermittelte Wert der Brennstoffgröße abgespeichert wird, beispielsweise in einem Speicher der Ermittlungseinrichtung. Dieser Wert steht nach dem Abschalten für das erneute Starten des Testbrenners und/oder des Hauptbrenners zur Verfügung. Er kann somit als Ausgangsgröße dienen, solange der Testbrenner noch keinen aktuelleren Wert für die Brennwertgröße ermittelt hat.

Vorteilhafte Ausgestaltungen der Erfindung ergeben sich aus den abhängigen Patentansprüchen sowie der Beschreibung. Die Beschreibung beschränkt sich auf wesentliche Merkmale der Erfindung. Die Zeichnung ist ergänzend heranzuziehen. Nachfolgend werden Ausführungsbeispiele der Erfindung anhand der beigefügten Zeichnung im Einzelnen erläutert. Es zeigen:
Figur 1 ein Blockschaltbild eines Ausführungsbeispiels einer Vorrichtung zur Bestimmung einer Brennwertgröße mit einem Testbrenner,
Figur 2 ein Blockschaltbild eines Ausführungsbeispiels einer gasbetriebenen Einrichtung mit einem Hauptbrenner,
Figur 3 eine schematische Darstellung der Temperatur T in Abhängigkeit vom Abstand z von einem Einlass in die Testbrennkammer der Vorrichtung nach Figur 1,
Figur 4 eine schematische Darstellung der Abhängigkeit des Ionisationssignals I von der Brennerleistung P des Testbrenners für verschiedene Luftzahlen λ und
Figur 5 eine schematische Darstellung der Abhängigkeit des Ionisationssignals I von der Brennerleistung P für verschiedene Brennwerte bzw. Zusammensetzungen des gasförmigen Brennstoffs.

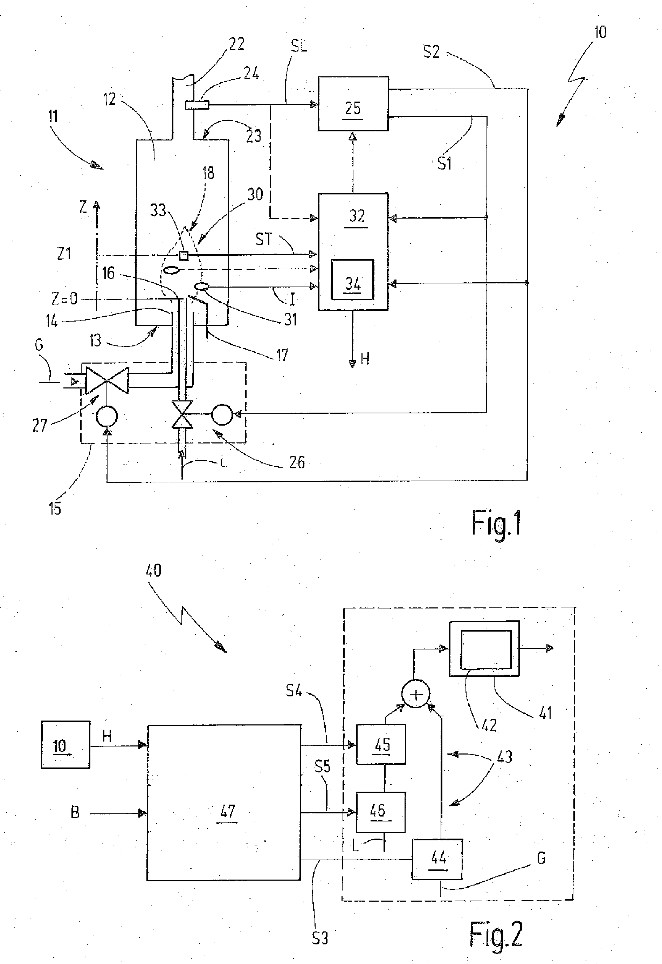

Figur 1 zeigt ein Ausführungsbeispiel einer Vorrichtung 10 zur Bestimmung einer Brennwertgröße H im Blockschaltbild. Die Brennwertgröße H gibt den Brennwert eines gasförmigen Brennstoffes G an. Bei dem gasförmigen Brennstoff G kann es sich beispielsweise um Biogas, Erdgas, Wasserstoff oder ein Gemisch aus mehreren der genannten Gasbestandteile handeln. Häufig ist die genaue Zusammensetzung des gasförmigen Brennstoffes G und mithin dessen Brennwert unbekannt. Die Vorrichtung 10 erlaubt die Bestimmung einer den Brennwert beschreibenden Brennwertgröße H.

Hierfür weist die Vorrichtung 10 einen Testbrenner 11 mit einer Testbrennkammer 12 auf. In die Testbrennkammer 12 mündet an einer ersten Seite 13 ein erster Einlass 14. Über den ersten Einlass 14 wird gasförmiger Brennstoff G über eine Testzuführeinrichtung 15 in die Brennkammer 12 eingeleitet.

An der ersten Seite 13 mündet außerdem ein zweiter Einlass 16 in die Testbrennkammer 12 ein, über den ein sauerstoffhaltiges Gas, beispielsgemäß Luft L, in die Testbrennkammer 12 über die Testzuführeinrichtung 15 eingeleitet wird. Die beiden Einlässe 14, 16 sind beim Ausführungsbeispiel koaxial zueinander angeordnet. Der zweite Einlass 16 ist dabei weiter von der ersten Seite 13 der Testbrennkammer 12 entfernt als der erste Einlass 14.

Alternativ zu dem Ausführungsbeispiel könnten der Brennstoff G und die Luft L auch in einem Mischer außerhalb der Testbrennkammer 12 gemischt werden.

Im Bereich des zweiten Einlasses 16 ist ein Zündmittel und beispielsgemäß eine Zündelektrode 17 angeordnet, um eine Testbrennerflamme 18 in der Testbrennkammer 12 erzeugen zu können. In Abwandlung zu dem hier dargestellten Ausführungsbeispiel könnte in der Testbrennkammer 12 auch ein Katalysator vorhanden sein, um eine Oxidation des Gemischs aus gasförmigen Brennstoff G und Luft L auszulösen.

Mit Abstand zur ersten Seite 13 führt aus der Testbrennkammer 12 ein Abgaskanal 22 heraus. Beim Ausführungsbeispiel ist die Mündung des Abgaskanals 22 auf der der ersten Seite 13 gegenüberliegenden zweiten Seite 23 der Testbrennkammer 12 angeordnet.

Im Abgaskanal 22 ist ein Luftzahlsensor 24 angeordnet. Der Luftzahlsensor 24 erzeugt ein Luftzahlsignal SL. Das Luftzahlsignal SL wird an eine Teststeuereinheit 25 übermittelt. Abhängig vom empfangenen Luftzahlsignal SL erzeugt die Teststeuereinheit 25 wenigstens ein Stellsignal S1, S2 zur Ansteuerung der Testzuführeinheit 15. Bei dem hier beschriebenen Ausführungsbeispiel wird durch die Teststeuereinheit 25 ein erstes Stellsignal S1 sowie ein zweites Stellsignal S2 für die Testzuführeinheit 15 erzeugt. Über das wenigstens eine Stellsignal S1, S2 kann der Anteil und/oder die Menge der Luft L und/oder des gasförmigen Brennstoffes G eingestellt werden, der in die Testbrennkammer 12 eingeleitet wird.

Bei dem Ausführungsbeispiel nach Figur 1 steuert das erste Stellsignal S1 ein erstes Stellmittel 26, beispielsweise ein steuerbares Proportionalventil, an, um die Testbrennkammer 12 zugeführte Menge an Luft L einzustellen. Mittels des zweiten Stellsignals S2 wird beispielsgemäß ein zweites Stellmittel 27, zum Beispiel ein steuerbares Proportionalventil, angesteuert, um die Menge an gasförmigem Brennstoff G einzustellen, die der Testbrennkammer 12 zugeführt wird. Auf diese Weise lässt sich der Anteil von Luft L bzw. von gasförmigem Brennstoff G sowie die Gesamtmenge des der Testbrennkammer 12 zugeführten Gasgemischs variabel einstellen. Alternativ zu dem Ausführungsbeispiel könnte die Menge an zugeführtem gasförmigem Brennstoff G oder an der zugeführten Luft L auch unveränderlich konstant vorgegeben sein, so dass lediglich die jeweils andere Komponente des Gemisches zur Veränderung der Anteile über ein Stellmittel 26 bzw. 27 einstellbar ist.

Alternativ oder zusätzlich zum steuerbaren Proportionalventil könnte das erste Stellmittel 26 auch ein steuerbares Gebläse oder ähnliches aufweisen.

In der Testbrennkammer 12 ist eine Brennwertsensoranordnung 30 vorhanden. Die Brennwertsensoranordnung 30 weist wenigstens einen Ionisationssensor 31 auf, der ein Ionisationssignal erzeugt und an eine Ermittlungseinheit 23 übermittelt. Beim Ausführungsbeispiel ist das Ionisationssignal durch den Ionisationsstrom I gebildet. Wie in Figur 1 schematisch veranschaulicht, können auch mehrere Ionisationssensoren 31 vorhanden sein, die jeweils ein separates Ionisationssignal I an die Ermittlungseinheit 32 übermitteln. Dadurch kann an verschiedenen Stellen in der Testbrennkammer 12 und insbesondere im Bereich der Testbrennerflamme 18 die Ionisation gemessen werden. Ein einziger Ionisationssensor 31, der insbesondere im Bereich der Testbrennerflamme 18 angeordnet ist, ist ausreichend.

Bei dem hier beschriebenen Ausführungsbeispiel gehört zu der Brennwertsensoranordnung 30 ferner wenigstens ein Temperatursensor 33. Vorzugsweise ist auch der Temperatursensor 33 im Bereich der Testbrennerflamme 18 in der Testbrennkammer 12 angeordnet. Der Temperatursensor 33 liefert ein Temperatursignal ST an die Ermittlungseinheit 32.

Wie in Figur 1 schematisch veranschaulicht, können der Ermittlungseinheit 32 bei einer bevorzugten Ausführungsform außerdem weitere Signale bzw. Größen zur Verfügung gestellt werden. Vorzugsweise werden eine oder mehrere der folgenden Signale an die Ermittlungseinheit übermittelt: das Luftzahlsignal SL und/oder das erste Stellsignal S1 und/oder das zweite Stellsignal S2.

Aus dem wenigstens einen Stellsignal S1, S2 kann die aktuell eingestellte Brennerleistung P des Testbrenners 11 ermittelt oder zumindest abgeschätzt werden. Da die Brennerleistung P den Ionisationsstrom I beeinflusst, ist es vorteilhaft, der Ermittlungseinheit 32 ein die Brennerleistung P beschreibendes Signal zuzuführen. Beispielsgemäß wird dies durch die Übermittlung des wenigstens einen Stellsignals S1, S2 erreicht. Alternativ hierzu kann die Brennerleistung P auch stets konstant gehalten werden, so dass der Ermittlungseinheit 32 die Brennerleistung P vorgegeben sein kann und nicht durch Übermittlung eines Signals bestimmt werden muss.

Aus den übermittelten Signale und beispielsgemäß dem Ionisationssignal I, dem Temperatursignal ST sowie der wenigstens einen Stellgröße S1, S2 ermittelt die Ermittlungseinheit 32 die Brennwertgröße H. Hierfür ist in einem Speicher 34 der Ermittlungseinheit 32 beispielsgemäß ein Kennfeld abgespeichert, anhand dem aus den Eingangssignalen I, T, S1, S2 die Brennwertgröße H bestimmt werden kann. Das Kennfeld kann im Speicher 34 in Form von einen oder mehreren Tabellen bzw. Matrizen abgespeichert sein.

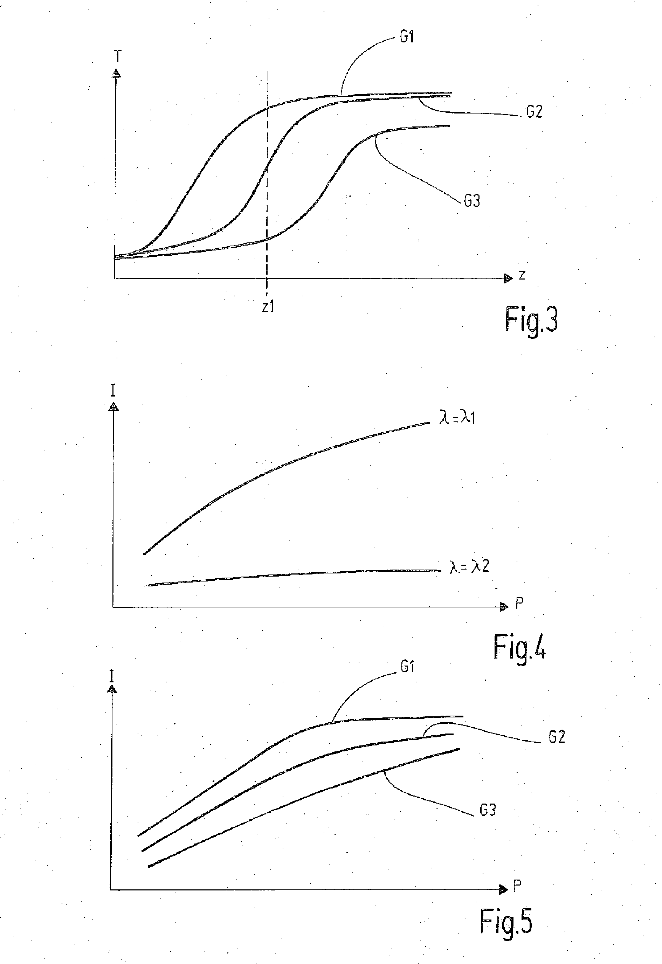

Anhand der Figuren 3 bis 5 wird die Funktionsweise der Vorrichtung 10 zur Bestimmung der Brennwertgröße H nachfolgend erläutert.

Zu Beginn des Betriebs wird die Testzuführeinheit 15 so eingestellt, dass eine vorgegebene Menge an gasförmigem Brennstoff G und Luft L in die Testbrennkammer 12 eingeleitet wird. Der Ausgangswert zur Einstellung der Testzuführeinheit 15 kann beispielsweise durch die wenigstens eine Stellgröße S1, S2 erfolgen, die beim vorhergehenden Betrieb der Vorrichtung 10 zuletzt verwendet wurde. Dieser Wert für die wenigstens eine Stellgröße S1, S2 kann im Speicher 34 der Ermittlungseinheit 32 abgespeichert sein und der Teststeuereinheit 25 bereitgestellt werden. Die Ausgangseinstellung der Testzuführeinheit 15 kann alternativ oder zusätzlich auch anhand der zuletzt ermittelten Brennwertgröße H erfolgen. Die jeweils aktuell bestimmte Brennwertgröße H kann hierzu im Speicher 24 während des Betriebs der Vorrichtung 10 abgespeichert werden.

Im Anschluss wird über die Zündelektrode 17 die Testbrennerflamme 18 gezündet. Über die Teststeuereinheit 25 wird die Testzuführeinheit 15 derart angesteuert, dass die dem Luftzahlsignal SL entsprechende Luftzahl λ einem Luftzahlsollwert entspricht. Dieser Luftzahlsollwert ist vorzugsweise größer oder gleich eins.

Wie in Figur 4 schematisch veranschaulicht, hängt der Ionisationsstrom I, der vom Ionisationssensor 31 gemessen wird, von der eingestellten Luftzahl λ ab. Die Werte des Ionisationsstroms I werden in der Ermittlungseinheit 32 insbesondere erst dann bewertet, wenn über die Teststeuereinheit 25 und die Testzuführeinrichtung 15 eine vorgegebene Luftzahl λ eingestellt wurde. Alternativ hierzu ist es auch möglich, Schwankungen der Luftzahl λ zuzulassen und der Ermittlungseinheit 32 das Luftzahlsignal SL zu übermitteln, so dass die jeweils aktuelle Luftzahl λ bei der Ermittlung der Brennwertgröße H berücksichtigt werden kann. Es ist ferner möglich, mehrere vorgegebene Luftzahlsollwerte zeitlich nacheinander einzustellen und bei jedem Luftzahlsollwerte eine Auswertung des wenigstens einen Sensorsignals der Brennwertsensoranordnung und insbesondere des Ionisationsstromes I uns des Temperatursignals ST unter Berücksichtigung der aktuellen Testbrennerleistung P durchzuführen und daraus die Brennwertgröße H zu ermitteln.

In Figur 3 ist schematisch die Abhängigkeit der Temperatur T vom Abstand z vom Fuß oder Kern der Flamme und beispielsgemäß vom zweiten Einlass 16 dargestellt. Dort ist zu erkennen, dass bei bestimmten Abständen z eine bessere Unterscheidbarkeit verschiedener Zusammensetzung G1, G2, G3 des gasförmigen Brennstoffs G möglich ist. Wie in Figur 3 schematisch veranschaulicht, wurde der Abstand z = z1 gewählt (siehe auch Figur 1), da in diesem Bereich die Unterschiede der Temperatur T für verschiedenen Zusammensetzungen G1, G2, G3 des gasförmigen Brennstoffes G deutlich unterscheidbar sind.

In Figur 5 ist der Ionisationsstrom I abhängig von der Leistung P des Testbrenners 12 schematisch veranschaulicht. Aus Figur 5 ist zu erkennen, dass sich für unterschiedliche Zusammensetzungen G1, G2, G3 des gasförmigen Brennstoffes G unterschiedliche Ionisationsströme I bei einer bekannten bzw. vorgegebenen Brennerleistung P des Testbrenners 11 ergeben.

Diese unterschiedlichen Zusammensetzungen G1, G2, G3 des gasförmigen Brennstoffes G haben unterschiedliche Brennwerte. In einem Kennfeld der Ermittlungseinheit 32 können daher den gemessenen Ionisationsströmen I und den gemessenen Temperaturen T gegebenenfalls unter Berücksichtigung der aktuellen Luftzahl λ sowie der aktuellen Brennerleistung P jeweils eine Brennwertgröße H zugeordnet werden. Wie erwähnt können die Luftzahl λ und die Brennerleistung P entweder vorgegeben auf einen bestimmten Wert eingestellt oder variabel sein. Im letzteren Fall können der Ermittlungseinheit 32 zur Berücksichtigung der aktuellen Luftzahl λ bzw. der aktuellen Brennerleistung P jeweils ein die Luftzahl λ bzw. die Brennerleistung P beschreibendes Signal übermittelt werden.

Die Vorrichtung 10 gemäß Figur 1 lässt sich vorteilhaft in einer gasbetriebenen Einrichtung 40 (Figur 2) mit einem Hauptbrenner 41, der eine Hauptbrennkammer 42 aufweist, einsetzen. Die gasbetriebene Einrichtung weist eine steuerbare Hauptzuführeinrichtung 43 auf. Die Hauptzuführeinrichtung 43 weist ein drittes Stellmittel 44 auf, mittels dem die Menge an gasförmigem Brennstoff G, die der Hauptbrennkammer 42 zugeführt wird, einstellbar ist. Ferner verfügt die Hauptzuführeinrichtung 43 über ein viertes Stellmittel 45 und/oder ein fünftes Stellmittel 46. Über das vierte Stellmittel 45 und/oder das fünfte Stellmittel 46 wird die Menge eines sauerstoffhaltigen Gases, beispielsgemäß Luft L, die der Hauptbrennkammer 42 zugeführt wird, eingestellt. Wie im Zusammenhang mit der Vorrichtung 10 nach Figur 1 beschrieben, können das dritte Stellmittel 44 und das vierte Stellmittel 45 durch ansteuerbare Proportionalventile gebildet sein. Alternativ oder zusätzlich kann zur Beeinflussung der Luftmenge ein fünftes Stellmittel 46, beispielsweise ein steuerbares Gebläse, vorgesehen sein. Der gasförmige Brennstoff G und die Luft L können wie in Figur 2 schematisch veranschaulicht außerhalb der Hauptbrennkammer 42 oder alternativ hierzu auch innerhalb der Hauptbrennkammer 42 gemischt werden.

Jedem Stellmittel 44, 45, 46 der Hauptzuführeinrichtung 43 ist ein entsprechendes Stellsignal S3, S4, S5 einer Hauptsteuereinheit 47 zugeordnet. Das wenigstens eine Stellsignal S3, S4, S5, das von der Hauptsteuereinheit 47 erzeugt wird, um die Hauptzuführeinheit 43 anzusteuern, wird abhängig von der Brennwertgröße H der Vorrichtung 10 ermittelt. Zusätzlich kann die Ermittlung des wenigstens einen Stellsignals S3, S4, S5 der Hauptsteuereinheit 47 von weiteren Signalen oder Parametern abhängen, beispielsweise von einem Bediensignal B, mittels dem der Betriebszustand bzw. die Brennerleistung des Hauptbrenners 41 vorgegeben wird. Das Bediensignal B kann beispielsweise durch eine Bedienperson oder auch automatisch von einem Regler oder einer Steuereinheit eingestellt werden. Außerdem können auch dem Hauptbrenner 41 Sensoren zugeordnet sein, deren Sensorsignale der Hauptsteuereinheit 47 übermittelt werden und die zur Ermittlung des wenigstens einen Stellsignals S3, S4, S5 herangezogen werden. Zum Beispiel kann auch in einem Abgaskanal des Hauptbrenners 41 ein Luftzahlsensor vorhanden sein, so dass über die Hauptsteuereinheit 47 das wenigstens eine Stellsignal S3, S4, S5 zur Erzielung einer vorgegebenen Luftzahl eingestellt wird.

In der Hauptsteuereinheit 47 kann zum Beispiel ein Kennfeld abgespeichert sein, das zumindest von der Brennwertgröße H abhängt und anhand dem sich jeweils ein entsprechender Wert für das von der Hauptsteuereinheit 47 erzeugte mindestens eine Stellsignal S3, S4, S5 ergibt. Das Kennfeld berücksichtigt beispielsgemäß auch das Bediensignal B als Eingangsgröße und/oder weitere Parameter, wie zum Beispiel ein Luftzahlsignal eines Luftzahlsensors im Abgaskanal des Hauptbrenners 41.

Es kann somit eine Steuerung bzw. Regelung des Hauptbrenners 41 angepasst an die Qualität bzw. den Brennwert des gasförmigen Brennstoffes G erfolgen.

Die ermittelte Brennwertgröße H kann auch für andere Anwendungen verwendet werden. Beispielsweise ist es auch möglich, einer Einrichtung zur Bestimmung des Energieverbrauchs die Brennwertgröße H des gasförmigen Brennstoffes G zu übermitteln. Dort wird der Energieverbrauch dann nicht zur unter Berücksichtigung der verbrauchten Gasmenge, sondern auch unter Berücksichtigung des einer bestimmten Gasmenge zugeordneten Brennwerts ermittelt. Diese Anwendung stellt einen weiteren eigenständigen, unabhängigen Aspekt der Erfindung dar.

Die Erfindung betrifft ein Verfahren und eine Vorrichtung zur Bestimmung einer den Brennwert eines gasförmigen Brennstoffes G beschreibenden Brennwertgröße H. Die Erfindung betrifft auch eine gasbetriebene Einrichtung 40, die ein erfindungsgemäßes Verfahren bzw. eine erfindungsgemäße Vorrichtung 10 verwendet. Die Vorrichtung 10 verfügt über einen Testbrenner 11 mit einer Testbrennkammer 12. Ein Luftzahlsensor 24 ist in einem Abgaskanal 22 des Testbrenners 11 angeordnet und misst ein Luftzahlsignal SL, das der Luftzahl λ des Abgases entspricht. Über eine Teststeuereinheit 25 wird abhängig vom empfangenen Luftzahlsignal SL wenigstens ein Stellsignal S1, S2 für eine Testzuführeinheit 15 erzeugt. Das wenigstens eine Stellsignal S1, S2 steuert die Menge und/oder den Anteil eines gasförmigen Brennstoffs G bzw. eines sauerstoffhaltigen Gases L, das der Testbrennkammer 12 zugeführt wird. In der Testbrennkammer 12 verbrennt bzw. oxidiert das Gemisch aus Brennstoff G und dem sauerstoffhaltigen Gas L. In der Brennkammer 12 ist eine Brennwertsensoranordnung 30 vorhanden. Zu dieser gehört zumindest ein Ionisationssensor 31 und vorzugsweise auch ein Temperatursensor 33. Das wenigstens eine Sensorsignal I bzw. T der Brennwertsensoranordnung wird einer Ermittlungseinheit 32 übermittelt. Dort wird vorzugsweise anhand eines Kennfeldes aus diesen Größen eine Brennwertgröße H ermittelt. Zur Ermittlung der Brennwertgröße H kann optional auch ein die Luftzahl λ beschreibendes Signal und/oder ein die Brennerleistung P des Testbrenners 11 beschreibendes Signal an die Ermittlungseinheit 32 übermittelt und berücksichtigt werden.

### Bezugszeichenliste:

- 10: Vorrichtung
- 11: Testbrenner
- 12: Testbrennkammer
- 13: erste Seite der Testbrennkammer
- 14: erster Einlass
- 15: Testzuführeinheit
- 16: zweiter Einlass
- 17: Zündelektrode
- 18: Testbrennerflamme

- 22: Abgaskanal
- 23: zweite Seite der Testbrennkammer
- 24: Luftzahlsensor
- 25: Teststeuereinheit
- 26: erstes Stellmittel
- 27: zweites Stellmittel

- 30: Brennwertsensoranordnung
- 31: Ionisationssensor
- 32: Ermittlungseinheit
- 33: Temperatursensor
- 34: Speicher

- 40: gasbetriebene Einrichtung
- 41: Hauptbrenner
- 42: Hauptbrennkammer
- 43: Hauptzuführeinrichtung
- 44: drittes Stellmittel
- 45: viertes Stellmittel
- 46: fünftes Stellmittel
- 47: Hauptsteuereinheit
- λ: Luftzahl

- B: Bediensignal
- G: gasförmiger Brennstoff
- G1: erste Zusammensetzung des Gasförmigen Brennstoffs
- G2: zweite Zusammensetzung des Gasförmigen Brennstoffs
- G3: dritte Zusammensetzung des Gasförmigen Brennstoffs
- H: Brennwertgröße
- I: Ionisationssignal
- S1: erstes Stellsignal
- S2: zweites Stellsignal
- S3: drittes Stellsignal
- S4: viertes Stellsignal
- S5: fünftes Stellsignal
- SL: Luftzahlsignal
- ST: Temperatursignal

## Patentansprüche

1. Vorrichtung zur Bestimmung einer Brennwertgröße (H) für den Brennwert eines gasförmigen Brennstoffes (G),
mit einer Testbrennkammer (12) und einem aus der Testbrennkammer (12) herausführenden Abgaskanal (22), in dem ein Luftzahlsensor (24) angeordnet ist,
mit einer steuerbaren Testzuführeinheit (15), mittels der die Menge des gasförmigen Brennstoffs (G) und/oder die Menge eines sauerstoffhaltigen Gases (L) eingestellt wird, die jeweils in die Testbrennkammer (12) geleitet wird,
mit einer Teststeuereinheit (25), der das Luftzahlsignal (SL) übermittelt wird und die abhängig vom Luftzahlsignal (SL) wenigstens ein Stellsignal (S1, S2) erzeugt, mittels dem die Testzuführeinheit (15) angesteuert wird, so dass die Luftzahl (λ) einem vorgegebenen Luftzahlsollwert entspricht,
mit einer in der Testbrennkammer (12) vorhandenen Brennwertsensoranordnung (30), die wenigstens einen Ionisationssensor (31) aufweist,
mit einer Ermittlungseinheit (32), der das Ionisationssignal (I) des Ionisationssensor (31) übermittelt wird, wobei die Ermittlungseinheit (32) abhängig vom Ionisationssignal (I) die Brennwertgröße (H) ermittelt, die den Brennwert des gasförmigen Brennstoffes (G) angibt.

2. Vorrichtung nach Anspruch 1,
**dadurch gekennzeichnet, dass** der Ermittlungseinheit (32) auch das Luftzahlsignal (SL) übermittelt wird und die Ermittlungseinheit (32) die Brennwertgröße (H) zusätzlich abhängig vom Luftzahlsignal (SL) ermittelt.

3. Vorrichtung nach Anspruch 1 oder 2,
**dadurch gekennzeichnet, dass** der Ermittlungseinheit (32) auch das wenigstens eine Stellsignal (S1, S2) übermittelt wird und die Ermittlungseinheit (32) die Brennwertgröße (H) zusätzlich abhängig vom Stellsignal (S1, S2) ermittelt.

4. Vorrichtung nach einem der vorhergehenden Ansprüche,
**dadurch gekennzeichnet, dass** die Brennwertsensoranordnung (30) wenigstens einen Temperatursensor (33) aufweist und das Temperatursignal (ST) des Temperatursensors (33) der Ermittlungseinheit (32) übermittelt wird, die Brennwertgröße (H) zusätzlich abhängig vom Temperatursignal (ST) ermittelt.

5. Vorrichtung nach einem der vorhergehenden Ansprüche,
**dadurch gekennzeichnet, dass** die Ermittlungseinheit (32) einen Speicher (34) aufweist, in dem eine Ermittlungsvorschrift zur Bestimmung der Brennwertgröße (H) abhängig vom Ionisationssignal (I) und wenigstens einem weiteren Parameter (T, SL, S1, S2) vorgegeben ist.

6. Vorrichtung nach einem der vorhergehenden Ansprüche,
**dadurch gekennzeichnet, dass** in der Testbrennkammer (12) eine Testbrennerflamme (18) erzeugt wird.

7. Vorrichtung nach einem der vorhergehenden Ansprüche,
**dadurch gekennzeichnet, dass** zur Bestimmung der Brennwertgröße (H) in der Ermittlungseinrichtung (32) das Temperatursignal (ST) und/oder das Ionisationssignal (I) bei einem vorgegebenen Wert oder mehreren vorgegebenen Werten für die Luftzahl (λ) erfasst wird.

8. Vorrichtung nach einem der vorhergehenden Ansprüche,
**dadurch gekennzeichnet, dass** mehrere Ionisationssensoren (31) an verschiedenen Stellen innerhalb der Testbrennkammer (12) angeordnet sind.

9. Gasbetriebene Einrichtung (40) mit einer steuerbaren Hauptzuführeinrichtung (43), mittels der die Menge eines gasförmigen Brennstoffs (G) und die Menge eines sauerstoffhaltigen Gases (L) eingestellt werden, die in eine Hauptbrennkammer (42) geleitet werden,
mit einer Vorrichtung (10) zur Bestimmung einer Brennwertgröße (H) nach einem der vorhergehenden Ansprüche,
und mit einer Hauptsteuereinheit (47), die abhängig von der Brennwertgröße (H) wenigstens ein Stellsignal (S3, S4, S5) zur Ansteuerung der Hauptzuführeinrichtung (43) erzeugt.

10. Gasbetriebene Einrichtung nach Anspruch 9,
**dadurch gekennzeichnet, dass** die Hauptsteuereinheit (47) dazu eingerichtet ist, die Zufuhr von gasförmigem Brennstoff (G) über die Hauptzuführeinheit (43) in die Hauptbrennkammer (42) erst dann freizugeben, wenn durch die Vorrichtung (10) zur Bestimmung einer Brennwertgröße (H) eine Brennwertgröße (H) übermittelt wurde.

11. Verfahren zur Bestimmung einer Brennwertgröße (H) für den Brennwert eines gasförmigen Brennstoffes (G) unter Verwendung einer Vorrichtung (10) mit einer Testbrennkammer (12) und einem aus der Testbrennkammer (12) herausführenden Abgaskanal (22), in dem ein Luftzahlsensor (24) angeordnet ist, mit einer steuerbaren Testzuführeinheit (15), mittels der die Menge des gasförmigen Brennstoffs (G) und/oder die Menge eines sauerstoffhaltigen Gases (L) eingestellt wird, die jeweils in die Testbrennkammer (12) geleitet wird, mit einer Teststeuereinheit (25), der das Luftzahlsignal (SL) übermittelt wird und die abhängig vom Luftzahlsignal (SL) wenigstens ein Stellsignal (S1, S2) für die Testzuführeinheit (15) erzeugt, mit einer in der Testbrennkammer (12) vorhandenen Brennwertsensoranordnung (30), die wenigstens einen Ionisationssensor (31) aufweist, und mit einer Ermittlungseinheit (32), der das Ionisationssignal (I) des Ionisationssensor (31) übermittelt wird, bei dem:
- die Testzuführeinheit (15) derart angesteuert wird, dass die Luftzahl (λ) einem vorgegebenen Luftzahlsollwert(λ) entspricht,
- die Ermittlungseinheit (32) abhängig vom Ionisationssignal (I) die Brennwertgröße (H) ermittelt, die den Brennwert des gasförmigen Brennstoffes angibt.

12. Verfahren nach Anspruch 11,
**dadurch gekennzeichnet, dass** zumindest der aktuell ermittelte Wert der Brennstoffgröße (H) abgespeichert wird.

13. Verfahren nach Anspruch 12,
**dadurch gekennzeichnet, dass** der abgespeicherte Wert der Brennstoffgröße (H) beim nächsten Starten der Verbrennung oder Oxidation in der Testbrennkammer (12) zur Einstellung der Menge des gasförmigen Brennstoffs (G) und/oder die Menge eines sauerstoffhaltigen Gases (L) verwendet wird, die jeweils in die Testbrennkammer (12) geleitet wird.
